(19) Europäisches Patentamt / European Patent Office / Office européen des brevets

(11) **EP 4 717 236 A1**

(12) **EUROPEAN PATENT APPLICATION**

(43) Date of publication:
01.04.2026 Bulletin 2026/14

(21) Application number: 25184067.4

(22) Date of filing: 19.06.2025

(51) International Patent Classification (IPC):
*A61F 5/56* *(2006.01)*

(52) Cooperative Patent Classification (CPC):
A61F 5/566

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 27.09.2024 CN 202411358993

(71) Applicant: Tong, Allen Sue-Lin
Foster City, California 94404 (US)

(72) Inventor: Tong, Allen Sue-Lin
Foster City, California 94404 (US)

(74) Representative: De Lorenzo, Danilo et al
Jacobacci & Partners S.p.A.
Piazza della Vittoria, 11
25122 Brescia (IT)

(54) **ADJUSTABLE ANTI-SNORING DEVICE**

(57) An adjustable anti-snoring device includes two side pieces, an upper curved structure, two connectors and a lower curved structure. Two ends of the upper curved structure are respectively connected to the two side pieces. One end of each of the two connectors is connected to one of the two side pieces, and each of the two connectors includes two blocks, two guiding rods and an adjusting screw. The two guiding rods are movably inserted into the two blocks. The adjusting screw is between the two guiding rods, and the adjusting screw is movably inserted into the two blocks. One end of the lower curved structure is connected to the other end of one of the two connectors, and the other end of the lower curved structure is connected to the other end of the other of the two connectors.

Fig. 1

## Description

## BACKGROUND

Technical Field

The present disclosure relates to an anti-snoring device. More particularly, the present disclosure relates to an adjustable anti-snoring device.

Description of Related Art

Snoring is a common sleeping phenomenon that not only disturbs others' sleep but may also be a symptom of sleep apnea. The cause of snoring is that the narrow respiratory tract or the insufficient muscle tone of the middle respiratory tract causes an air-flow resistance through the respiratory tract to increase during breathing, and the soft tissue around the respiratory tract is vibrated to produce an abnormal sound. A vibration force produced by seriously snoring may cause damage to the carotid artery, and cause the muscle of the middle respiratory tract to be increasingly slack. Therefore, the patency of the respiratory tract will be influenced, and cause a more seriously vicious circle of snoring.

For people with snoring, the most common improvement method is to wear an anti-snoring device, which is a non-invasive device. The mandible of a user can be adjusted forward and downward by wearing the anti-snoring device, the radix linguae can be simultaneously moved forward and downward to increase a space of the respiratory tract behind the tongue so as to reduce the snoring and the sleep apnea. The working principle of a conventional anti-snoring device is to give a present movement position to the user's mandible so as to move the radix linguae and expand the respiratory tract. However, the required expanding space of the respiratory tracts for each user is different, and a single-design anti-snoring device cannot meet the requirement of everyone. As a result, some users opt for customized anti-snoring devices to meet individual requirements. Nevertheless, when the requirement for adjusting the mandible position of the user changes, the customized anti-snoring device should be re-customized. If adjustments are needed frequently, multiple customized anti-snoring devices must be made, which can be a significant expense for the user.

It is necessary to develop an anti-snoring device that can solve the problems of snoring, can adjust the expanding degree of the respiratory tract according to the users' requirement, and is comfortable to wear.

## SUMMARY

According to one aspect of the present disclosure, an adjustable anti-snoring device includes two side pieces, an upper curved structure, two connectors and a lower curved structure. Two ends of the upper curved structure are respectively connected to the two side pieces. One end of each of the two connectors is connected to one of the two side pieces, and each of the two connectors includes two blocks, two guiding rods and an adjusting screw. Two guiding rods are movably inserted into the two blocks and parallel to each other. The adjusting screw is between the two guiding rods and parallel to the two guiding rods, wherein the adjusting screw is movably inserted into the two blocks. The lower curved structure includes a recess so as to accommodate a lower dental arch of a user. The lower curved structure is disposed below the upper curved structure, one end of the lower curved structure is connected to the other end of one of the two connectors, and the other end of the lower curved structure is connected to the other end of the other of the two connectors. An extending distance is existed between the two blocks by rotating the adjusting screw, the extending distance is 0.25 mm to 9.00 mm, an extending direction of the extending distance is parallel to an axial direction of the adjusting screw, and the extending direction is parallel to a sliding direction of a temporomandibular joint of the user.

In one example, the upper curved structure can include a recess so as to accommodate an upper dental arch of the user.

In one example, a depth of the recess of the upper curved structure can be 6 mm to 16 mm, and a depth of the recess of the lower curved structure can be 6 mm to 16 mm.

In one example, the adjustable anti-snoring device can further include a curved element. Two ends of the curved element are respectively connected to the two side pieces and corresponding to the upper curved structure, and the curved element is closer to a labial side of the user than the upper curved structure to the labial side of the user.

In one example, the adjustable anti-snoring device can further include two tubular structures. Each of the two tubular structures is disposed on an outer side of one of the two side pieces, and the two ends of the curved element are disposed in the two tubular structures, respectively.

In one example, the two ends of the curved element can be movably inserted into the two tubular structures, respectively.

In one example, a thickness of each of the two tubular structures can be 1.0 mm to 3.0 mm.

In one example, the upper curved structure can include an elastic material, the lower curved structure can include the elastic material, and each of the two side pieces can include the elastic material.

In one example, the elastic material can be a medical grade silicone, a latex or a natural rubber.

In one example, a material of each of the two connectors can include a titanium, an iron, a nickel, a cobalt, a chromium or a combination thereof.

**BRIEF DESCRIPTION OF THE DRAWINGS**

The present disclosure can be more fully understood by reading the following detailed description of the embodiment, with reference made to the accompanying drawings as follows:

Fig. 1 is a schematic view of an adjustable anti-snoring device according to one embodiment of the present disclosure.

Fig. 2 is a schematic view of using the adjustable anti-snoring device according to Fig. 1.

Fig. 3 is a cephalometric X-ray of a user.

Fig. 4 is a schematic view of an adjustable anti-snoring device according to another embodiment of the present disclosure.

Fig. 5 is a schematic view of an adjustable anti-snoring device according to still another embodiment of the present disclosure.

Fig. 6 is a diagram of the environmental sound level of the user sleeping at night in Comparative example 1.

Fig. 7 is a diagram of the environmental sound level of the user sleeping at night in Example 1.

Fig. 8 is a diagram of comparing the average values of maximum snoring sound level of the user in Example 1 and the user in Comparative example 1.

**DETAILED DESCRIPTION**

The present disclosure will be further exemplified by the following specific embodiments. However, the embodiments can be applied to various inventive concepts and can be embodied in various specific ways. The specific embodiments are only for the purposes of description, and are not limited to these practical details thereof.

The present disclosure provides an adjustable anti-snoring device that can be structurally adjusted according to a user's requirement of expanding the respiratory tract to help the user to adjust a corresponding angle and a relative position of the upper dental arch to the lower dental arch. Therefore, the snoring can be efficiently reduced, and the wearing comfort for the user can be simultaneously considered.

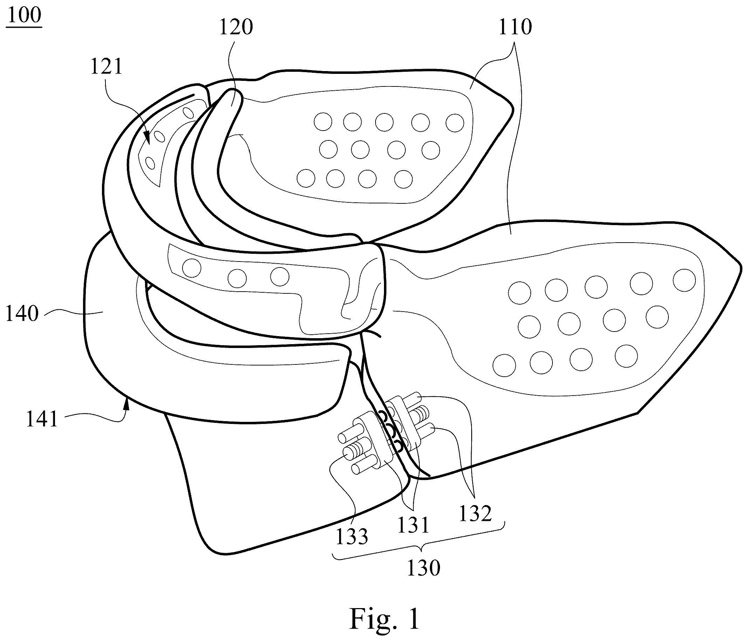

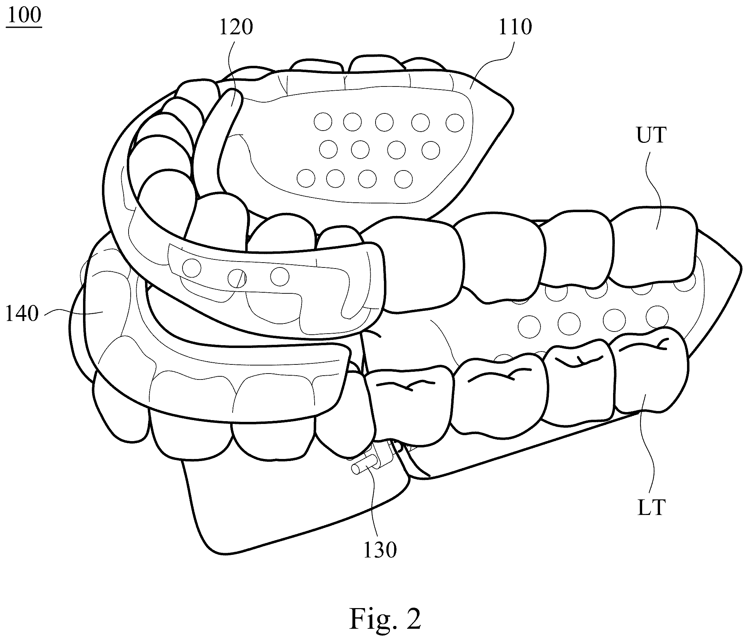

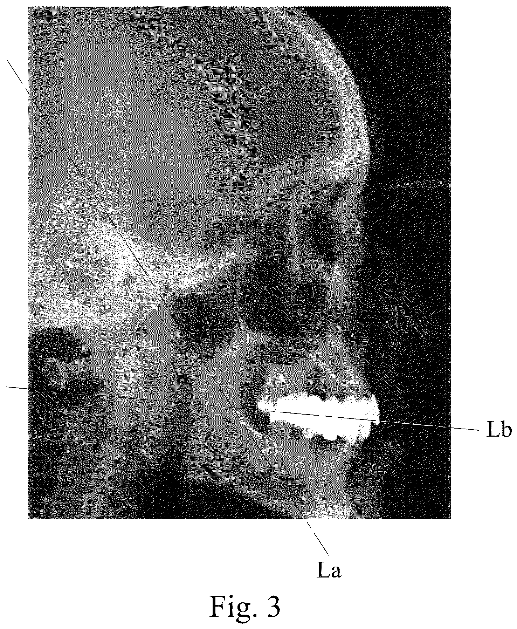

Reference is made to Fig. 1, Fig, 2 and Fig. 3, Fig. 1 is a schematic view of an adjustable anti-snoring device 100 according to one embodiment of the present disclosure, Fig. 2 is a schematic view of using the adjustable anti-snoring device 100 according to Fig. 1, and Fig. 3 is a cephalometric X-ray of a user. In Fig. 1, the adjustable anti-snoring device 100 of the present disclosure includes two side pieces 110, an upper curved structure 120, two connectors 130 and a lower curved structure 140, wherein two ends of the upper curved structure 120 are respectively connected to the two side pieces 110. One end of each of the two connectors 130 is connected to one of the two side pieces 110, the lower curved structure 140 is disposed below the upper curved structure 120, one end of the lower curved structure 140 is connected to the other end of one of the two connectors 130, and the other end of the lower curved structure 140 is connected to the other end of the other of the two connectors 130.

Specifically, the two side pieces 110 can abut the user's two ends of the lingual gingiva of an upper dental arch UT and the lingual gingiva of a lower dental arch LT. Therefore, the positioning stability in the user's mouth of the adjustable anti-snoring device 100 can be enhanced, and it is favorable for improving the anti-snoring effect of the adjustable anti-snoring device 100.

Furthermore, the upper curved structure 120 can include a recess 121 so as to accommodate the upper dental arch UT of the user. A lingual-side of the recess 121 is configured to abut a front lingual gingiva of the upper dental arch UT of the user, so that a labial-side and the lingual-side of the recess 121 can be away from the upper dental arch UT of the user to reduce the discomfort caused by the recess 121 pressing the upper dental arch UT of the user. Furthermore, the dentition shape change of the upper dental arch UT can be avoided, and the dentition shape change is caused by an improper pressing force on the upper dental arch UT of the user via the recess 121.

Each of the two connectors 130 includes two blocks 131, two guiding rods 132 and an adjusting screw 133, the two guiding rods 132 are movably inserted into the two blocks 131 and parallel to each other, the adjusting screw 133 is between the two guiding rods 132 and parallel to the two guiding rods 132, and the adjusting screw 133 is movably inserted into the two blocks 131.

The lower curved structure 140 includes a recess 141 so as to accommodate the lower dental arch LT of the user. Specifically, a lingual-side of the recess 141 is configured to abut a front lingual gingiva of the lower dental arch LT of the user, so that a labial-side and the lingual-side of the recess 141 can be away from the lower dental arch LT of the user to reduce the discomfort caused by the recess 141 pressing the lower dental arch LT of the user. Furthermore, the dentition shape change of the lower dental arch LT can be avoided, and the dentition shape change is caused by an improper pressing force on the lower dental arch LT of the user via the recess 141.

An extending distance is existed between the two blocks 131 by rotating the adjusting screw 133, an extending direction of the extending distance is parallel to an axial direction of the adjusting screw 133, and the extending direction is parallel to a sliding direction of a temporomandibular joint La of the user. In detail, a dental occlusion data of the user can be obtained via a digital articulator and used with the cephalometric X-ray of the user (shown in Fig. 3) obtained by a cephalometric analysis. Therefore, the sliding direction of the temporomandibular joint La and a dentition direction Lb can be obtained, and a position of the connectors 130 can be determined according to an intersection angle of the sliding direction of the temporomandibular joint La and the dentition direction Lb, wherein the center of the adjusting screw 133 of each of the connectors 130 is located in the dentition direction Lb, and an axial direction of the adjusting screw 133 of each of the connectors 130 is parallel to the sliding direction of the temporomandibular joint La.

Moreover, the extending distance existed between the two blocks 131 can be changed by rotating the adjusting screw 133. When the extending distance becomes larger, a distance between one end of the lower curved structure 140 and one of the side pieces 110 is increased. When the extending distance becomes smaller, the distance between the end of the lower curved structure 140 and the one of the side pieces 110 is decreased. A relative position of the end of the lower curved structure 140 to the one of the side pieces 110 of the adjustable anti-snoring device 100 can be adjusted by adjusting the adjusting screw 133 of each of the connectors 130, so that a relative position of the upper curved structure 120 to the lower curved structure 140 is changed. Specifically, the cause of the snoring is that the middle respiratory tract is narrow to cause a turbulent airflow of breathing, and the turbulent airflow vibrates the soft tissue around the respiratory tract to cause snoring. When the user wears the adjustable anti-snoring device 100, the relative position of the upper dental arch UT to the lower dental arch LT is changed so as to change the relative position of the maxilla to the mandible of the user. When the mandible of the user is moved forward and downward, the radix linguae can be simultaneously moved forward and downward to expand a space of the respiratory tract to reduce the snoring of the user.

Furthermore, the extending distance between the two blocks 131 is 0.25 mm to 9.00 mm. Preferably, the extending distance between the two blocks 131 can be 0.25 mm to 4.00 mm, so that it can be ensured that a distance between the labial-side of the recess 121 and the upper dental arch UT of the user is larger than 0.8 mm, and a distance between the labial-side of the recess 141 and the lower dental arch LT of the user is larger than 0.8 mm. Therefore, it is favorable for preventing the detention of the upper dental arch UT and the detention of the lower dental arch LT from being pressed by the labial-side and the lingual-side of the recess 121 and by the labial-side and the lingual-side of the recess 141 so as to reduce the risks of the dentition shape change of the upper dental arch UT and the lower dental arch LT, discomfort and the injury to the dental pulp tissue. Therefore, the wearing comfort of the adjustable anti-snoring device 100 can be improved, and the wearing stability in the user's mouth of the adjustable anti-snoring device 100 can be enhanced.

Further, a depth of the recess 121 of the upper curved structure 120 can be 6 mm to 16 mm, and a depth of the recess 141 of the lower curved structure 140 can be 6 mm to 16 mm. Preferably, the depth of the recess 121 of the upper curved structure 120 can be 8 mm to 10 mm, and the depth of the recess 141 of the lower curved structure 140 can be 8 mm to 10 mm. Therefore, it can be ensured that the space in the adjustable anti-snoring device 100 is enough to accommodate the upper dental arch UT and the lower dental arch LT of the user. Moreover, it can be ensured that the contacting areas of the labial-side of the recess 121 and the labial-side of the recess 141 are enough to the front lingual gingiva of the upper dental arch UT and the lower dental arch LT of the user. Therefore, the wearing stability in the user's mouth of the adjustable anti-snoring device 100 can be enhanced.

Furthermore, the upper curved structure 120 can include an elastic material, the lower curved structure 140 can include the elastic material, and each of the two side pieces 110 can include the elastic material. Therefore, the oppression in the user's mouth caused by the adjustable anti-snoring device 100 can be reduced, and the wearing comfort of the adjustable anti-snoring device 100 can be improved. Moreover, the adjustable anti-snoring device 100 can prevent the user from the teeth enamel damage caused by teeth grinding, and can ease the discomfort of the oral muscle soreness and the pain of the temporomandibular joint.

As the adjustable anti-snoring device 100 needs to be worn during daily sleep, and daily usage is essential to effectively reduce snoring. According to the above, it is estimated that the user wears the adjustable anti-snoring device 100 for over 200 hours each month. Therefore, the elastic material of the adjustable anti-snoring device 100 is selected with considering the safety and the durability. Based on the considering, a better choice of the elastic material can be a medical grade silicone, a latex or a natural rubber. Preferably, the elastic material can be the medical grade silicone, it is favorable for the durability of the adjustable anti-snoring device 100 in dry and humid environments, and the medical grade silicone has the high bio-compatibility to avoid undesirable influences on the human health.

Moreover, the upper curved structure 120 and the lower curved structure 140 of the adjustable anti-snoring device 100 can be manufactured based on a digital data of the user's oral cavity, and match the shape of the user's oral cavity. Therefore, the fit of the adjustable anti-snoring device 100 to the user's requirement can be improved. In a manufacturing method of the adjustable anti-snoring device 100, the digital data of the user's oral cavity can be obtained by oral scanning, the three dimensional views of the upper curved structure 120 and the lower curved structure 140 can be produced via a computer-aided design software, and the upper curved structure 120 and the lower curved structure 140 can be printed via a 3-D printer. With considering the process convenience of the 3-D printer, the elastic material can be the medical grade silicone or a stereolithographic medical grade silicone.

The adjustable anti-snoring device 100 needs to be worn in the user's mouth for over 5 hours each day, so that the connectors 130 should be exposed to a humid environment for a long time, and the connectors 130 needs to have the good mechanical strength, the corrosion resistance, the low toxicity and the bio-compatibility. Therefore, the connectors 130 of the adjustable anti-snoring device 100 of the present disclosure can be made of a metal material to provide the mechanical strength. In detail, a material of each of the two connectors 130 can include a titanium, an iron, a nickel, a cobalt, a chromium or a combination thereof. Furthermore, each of the two connectors 130 can include the titanium to increase the elasticity, and include the chromium to improve the corrosion resistance. Therefore, the durability and the reliability of the connectors 130 can be improved, and it is favorable for the stability of the adjustable anti-snoring device 100.

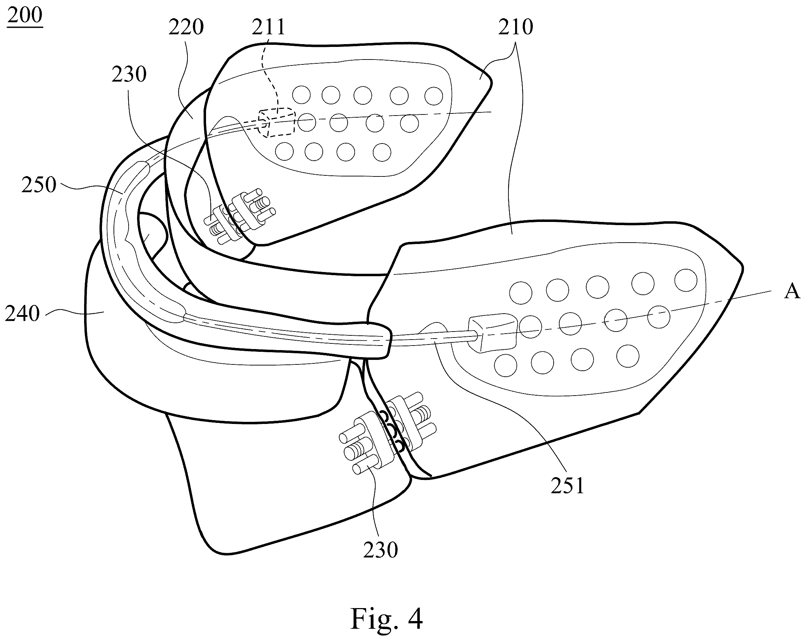

Reference is made to Fig. 4, which is a schematic view of an adjustable anti-snoring device 200 according to another embodiment of the present disclosure. In fig. 4, the adjustable anti-snoring device 200 of the present disclosure includes two side pieces 210, an upper curved structure 220, two connectors 230, a lower curved structure 240 and a curved element 250, wherein the structures, positions and the connection relationships of the two side pieces 210, the two connectors 230 and the lower curved structure 240 are similar to the structures, positions and the connection relationships of the two side pieces 110, the two connectors 130 and the lower curved structure 140, and the details will not be repeated here. The difference between the adjustable anti-snoring device 200 and the adjustable anti-snoring device 100 is that the upper curved structure 220 of the adjustable anti-snoring device 200 is sheet-like arc-shaped. Moreover, the adjustable anti-snoring device 200 further includes the curved element 250. Two ends of the curved element 250 are respectively connected to the two side pieces 210 and corresponding to the upper curved structure 220, and the curved element 250 is closer to a labial side of a user than the upper curved structure 220 to the labial side of the user. In detail, a distance is exited between the upper curved structure 220 and the curved element 250, when the user wears the adjustable anti-snoring device 200, the upper dental arch (not illustrated) of the user is between the upper curved structure 220 and the curved element 250. When the upper curved structure 220 abuts a front lingual gingiva of the upper dental arch of the user, so that an appropriate distance is kept between the curved element 250 and a tooth surface of the upper dental arch of the user to avoid the dentition shape change caused by an improper pressing force on the dentition of the upper dental arch of the user via the curved element 250.

Furthermore, the curved element 250 can include a bracket 251, the bracket 251 passes through the curved element 250 and corresponds to an arc axis A of the curved element 250. Each of the two side pieces 210 can further include a fixing base 211, the fixing base 211 is disposed on an outer side of each of the two side pieces 210, and each of two ends of the bracket 251 is fixed in the fixing base 211. A diameter of the bracket 251 can be 1.0 mm to 1.5 mm. Therefore, the structural stability of the curved element 250 can be improved.

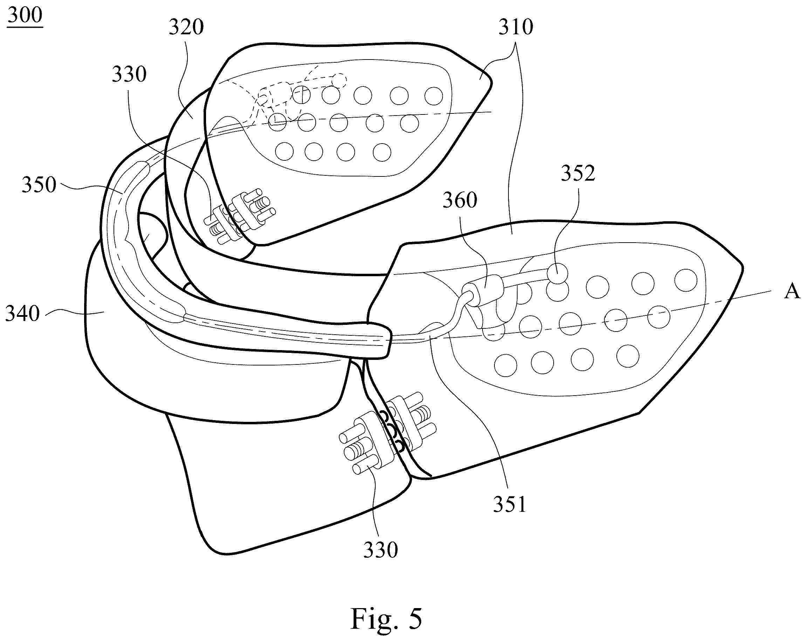

Reference is made to Fig. 5, which is a schematic view of an adjustable anti-snoring device 300 according to still another embodiment of the present disclosure. In Fig. 5, the adjustable anti-snoring device 300 of the present disclosure includes two side pieces 310, an upper curved structure 320, two connectors 330, a lower curved structure 340 and a curved element 350. The structures, positions and the connection relationships in the adjustable anti-snoring device 300 are similar to the structures, positions and the connection relationships in the adjustable anti-snoring device 200, and the details will not be repeated here. The difference between the adjustable anti-snoring device 300 and the adjustable anti-snoring device 200 is that the adjustable anti-snoring device 300 can further include two tubular structures 360. Each of the two tubular structures 360 is disposed on an outer side of one of the two side pieces 310, and the two ends of the curved element 350 are disposed in the two tubular structures 360, respectively. Moreover, the two ends of the curved element 350 are movably inserted into the two tubular structures 360, respectively. Therefore, the relative position of the curved element 350 to the upper curved structure 320 can be adjusted, and an appropriate distance between the curved element 350 and a tooth surface of the upper dental arch (not illustrated) of the user is kept according to the user's requirement so as to reduce the discomfort caused by the curved element 350 pressing the dentition of the upper dental arch of the user, and it is favorable for keeping the space stability of the expanded respiratory tract of the user.

In detail, the curved element 350 of the adjustable anti-snoring device 300 can include a bracket 351, the bracket 351 passes through the curved element 350 and corresponds to an arc axis A of the curved element 350. The bracket 351 is movably inserted into the two tubular structures 360, respectively. Therefore, the adjustment convenience of the curved element 350 can be improved.

Moreover, two ends of the bracket 351 of the adjustable anti-snoring device 300 can further include two spherical elements 352, one of the two spherical elements 352 is disposed at one end of the bracket 351, and the other of the two spherical elements 352 is disposed at the other end of the bracket 351. Therefore, any end of the bracket 351 is prevented from disengaging from one of the two tubular structures 360, and the reliability of the adjustable anti-snoring device 300 can be improved.

Furthermore, a thickness of each of the two tubular structures 360 can be 1.0 mm to 3.0 mm. Preferably, the thickness of each of the two tubular structures 360 can be 1.5 mm to 3.0 mm. Therefore, it is favorable for keeping the structural stability and the reliability of the adjustable anti-snoring device 300.

Further, a material of each of the two tubular structures 360 can include a titanium, an iron, a nickel, a cobalt, a chromium or a combination thereof. In detail, each of the two tubular structures 360 can include the titanium to increase the elasticity, and it is favorable for the structural stability and the durability of the curved element 350 being inserted into the tubular structures 360.

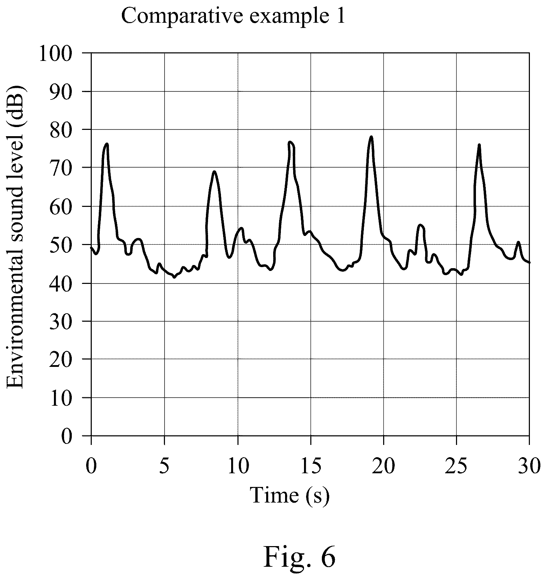

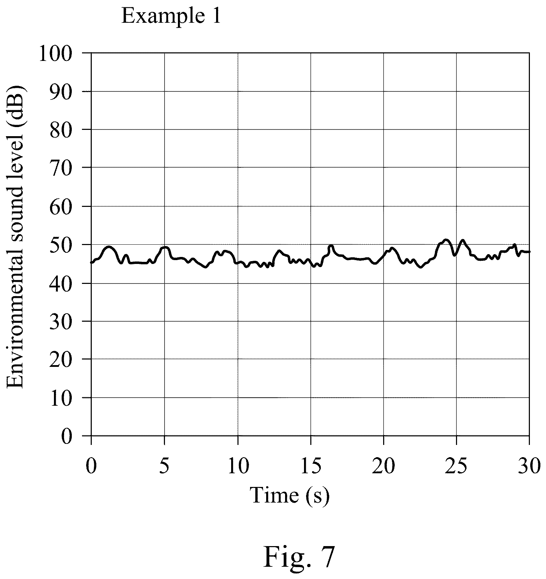

In order to prove that the snoring of a user can be reduced by wearing an adjustable anti-snoring device of the present disclosure, a user in Example 1 worn the adjustable anti-snoring device of the present disclosure, a user in Comparative example 1 did not wear the adjustable anti-snoring device of the present disclosure and any other anti-snoring device, and the average values of maximum snoring sound level during sleeping of the user in Example 1 and the user in Comparative example 1 were measured. The measurement was performed via a sound recording device that was placed 1 meter away from the user's head, and an environmental sound level of the user sleeping at night was recorded for 1 hour after the user falls asleep for 1.5 hours. The environmental sound level included a background noise level and a snoring sound level. Reference is made to Fig. 6 and Fig. 7, Fig. 6 is a diagram of the environmental sound level of the user sleeping at night in Comparative example 1, and Fig. 7 is a diagram of the environmental sound level of the user sleeping at night in Example 1. Fig. 6 and Fig. 7 are raw data taken from 30 seconds of the environmental sound level that are continuously recorded for 1 hour. In Fig. 6 and Fig. 7, the raw data of the environmental sound level are periodic peak diagrams, wherein the wave trough is the period between two snoring peaks, the sound level of the wave trough can be regarded as the background noise level, the wave peak appears during snoring, the sound level of the wave peak can be regarded as the maximum snoring sound level plus the background noise level, and the sound level of the wave peak is the maximum environmental sound level during snoring. The statistics of the above data is performed, an average environmental sound level ($L_2$) is obtained by averaging all the environmental sound levels, and the average value of maximum environmental sound levels during snoring ($L_1$) is obtained by averaging all the maximum environmental sound levels. According to the environmental sound level correction formula, the average value of maximum snoring sound level is obtained by deducing the average value of maximum environmental sound levels during snoring ($L_1$) from the environmental sound level ($L_2$), and the environmental sound level correction formula is as below:

$$L = 10\log\left(10^{0.1L_1} - 10^{0.1L_2}\right)$$

.

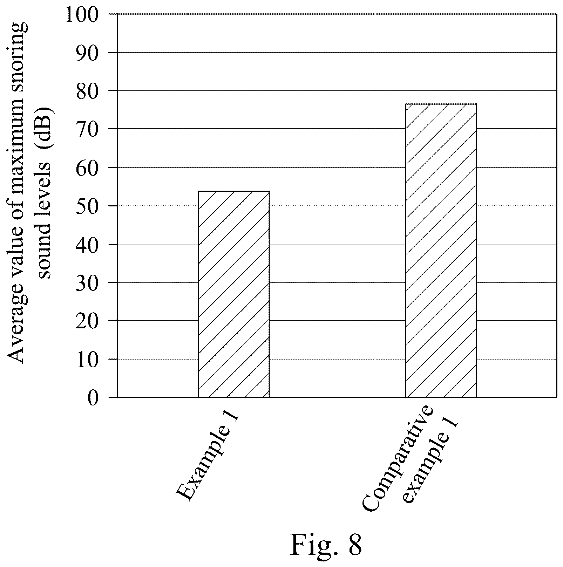

Reference is made to Fig. 8, which is a diagram of comparing the average values of maximum snoring sound level of the user in Example 1 and the user in Comparative example 1. In Fig. 8, the average value of maximum snoring sound level of the user in Example 1 is about 53 decibels, and the average value of maximum snoring sound level of the user in Comparative example 1 is about 76 decibels. According to the above data, the average value of maximum snoring sound level of the user in Example 1 is smaller than the average value of maximum snoring sound level of the user in Comparative example 1. It can prove that the adjustable anti-snoring device of the present disclosure can change the relative position of the upper dental arch to the lower dental arch of the user by adjusting the relative position of the upper curved structure to the lower curved structure, and it is favorable for expanding the space of the respiratory tract of the user to reduce the snoring.

In conclusion, the adjustable anti-snoring device of the present disclosure has an adjustable mechanism design. The relative position of the upper curved structure to the lower curved structure can be adjusted by adjusting the adjusting screw of each of the connectors according to the user's requirement. When the user wears the adjustable anti-snoring device, the relative position of the upper dental arch to the lower dental arch is changed to expand the space of the respiratory tract of the user, and the snoring can be eased. The extending distance of the adjusting screw is parallel to the sliding direction of the temporomandibular joint of the user, the upper curved structure can be designed to include an elastic material, and the lower curved structure can be designed to include the elastic material, so that the foreign-body-sensation of wearing the adjustable anti-snoring device can be reduced and the comfort of use can be improved. Furthermore, the discomfort of the oral muscle soreness and the pain of the temporomandibular joint can be eased.

**Claims**

1. An adjustable anti-snoring device (100), **characterized in** comprising:

   two side pieces (110);
   an upper curved structure (120), wherein two ends of the upper curved structure (120) are respectively connected to the two side pieces (110);
   two connectors (130), wherein one end of each of the two connectors (130) is connected to one of the two side pieces (110), and each of the two connectors (130) comprises:

two blocks (131);

two guiding rods (132) movably inserted into the two blocks (131) and parallel to each other; and

an adjusting screw (133) between the two guiding rods (132) and parallel to the two guiding rods (132), wherein the adjusting screw (133) is movably inserted into the two blocks (131); and

a lower curved structure (140) comprising a recess (141) so as to accommodate a lower dental arch (LT) of a user, wherein the lower curved structure (140) is disposed below the upper curved structure (120), one end of the lower curved structure (140) is connected to the other end of one of the two connectors (130), and the other end of the lower curved structure (140) is connected to the other end of the other of the two connectors (130);

wherein an extending distance is existed between the two blocks (131) by rotating the adjusting screw (133), the extending distance is 0.25 mm to 9.00 mm, an extending direction of the extending distance is parallel to an axial direction of the adjusting screw (133), and the extending direction is parallel to a sliding direction of a temporomandibular joint of the user.

2. The adjustable anti-snoring device (100) of claim 1, wherein the upper curved structure (120) comprises a recess (121) so as to accommodate an upper dental arch (UT) of the user.

3. The adjustable anti-snoring device (100) of claim 1-2, wherein a depth of the recess (121) of the upper curved structure (120) is 6 mm to 16 mm, and a depth of the recess (141) of the lower curved structure (140) is 6 mm to 16 mm.

4. The adjustable anti-snoring device (200) of claim 1-3, further comprising a curved element (250), wherein two ends of the curved element (250) are respectively connected to the two side pieces (210) and corresponding to the upper curved structure (220), and the curved element (250) is closer to a labial side of the user than the upper curved structure (220) to the labial side of the user.

5. The adjustable anti-snoring device (300) of claim 1-4, further comprising two tubular structures (360), wherein each of the two tubular structures (360) is disposed on an outer side of one of the two side pieces (310), and the two ends of the curved element (350) are disposed in the two tubular structures (360), respectively.

6. The adjustable anti-snoring device (300) of claim 1-5, wherein the two ends of the curved element (350) are movably inserted into the two tubular structures (360), respectively.

7. The adjustable anti-snoring device (300) of claim 1-6, wherein a thickness of each of the two tubular structures (360) is 1.0 mm to 3.0 mm.

8. The adjustable anti-snoring device (100) of claim 1-7, wherein the upper curved structure (120) comprises an elastic material, the lower curved structure (140) comprises the elastic material, and each of the two side pieces (110) comprises the elastic material.

9. The adjustable anti-snoring device (100) of claim 1-8, wherein the elastic material is a medical grade silicone, a latex or a natural rubber.

10. The adjustable anti-snoring device (100) of claim 1-9, wherein a material of each of the two connectors (130) comprises a titanium, an iron, a nickel, a cobalt, a chromium or a combination thereof.

Fig. 1

Fig. 2

Fig. 3

Fig. 4

Fig. 5

EP 4 717 236 A1

Comparative example 1

Fig. 6

Example 1

Fig. 7

Fig. 8

**EUROPEAN SEARCH REPORT**

Europäisches
Patentamt
European
Patent Office
Office européen
des brevets

**Application Number**

EP 25 18 4067

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/272773 A1 (RICO HECTOR [US] ET AL) 1 October 2015 (2015-10-01) * the whole document * ----- | 1-10 | INV. A61F5 |
| X | CA 2 996 663 A1 (YOUSEFIAN JOSEPH [US]) 2 March 2017 (2017-03-02) * the whole document * ----- | 1-10 | |

**TECHNICAL FIELDS SEARCHED (IPC)**

A61F

The present search report has been drawn up for all claims

| Place of search | Date of completion of the search | Examiner |
|---|---|---|
| Munich | 24 October 2025 | Louchet, Nicolas |

EPO FORM 1503 03.82 (P04C01)

## ANNEX TO THE EUROPEAN SEARCH REPORT
## ON EUROPEAN PATENT APPLICATION NO.

EP 25 18 4067

This annex lists the patent family members relating to the patent documents cited in the above-mentioned European search report.
The members are as contained in the European Patent Office EDP file on
The European Patent Office is in no way liable for these particulars which are merely given for the purpose of information.

24-10-2025

| Patent document cited in search report | Publication date | Patent family member(s) | | Publication date |
|---|---|---|---|---|
| US 2015272773 A1 | 01-10-2015 | NONE | | |
| CA 2996663 A1 | 02-03-2017 | CA | 2996663 A1 | 02-03-2017 |
| | | US | 2017056236 A1 | 02-03-2017 |
| | | WO | 2017035531 A1 | 02-03-2017 |

EPO FORM P0459

For more details about this annex : see Official Journal of the European Patent Office, No. 12/82